# EUROPEAN PATENT APPLICATION

(11) **EP 1 310 794 A2**
(43) Date of publication of application: **14.05.2003**
(21) Application number: 02445087.6
(22) Date of filing: 27.06.2002
(51) Int. Cl.: G01N 33/543

(54) **Antibody testing method and antigen microarray**

(30) Priority: 09.11.2001 JP 2001345132
(71) Applicant: Gifu University, Gifu-shi, Gifu 501-1193 (JP)
(72) Inventor: Ezaki, Takayuki, Gifu-shi, Gifu 500-8454 (JP)
(74) Representative: Akerman, Marten Lennart

(57) **Abstract**

A method for testing whether or not an antibody that binds with any of a plurality of target antigens is contained in a test sample. In this method, a test sample is contacted with an antigen microarray (100; 100a) having spots of at least eight types of target antigens. In the case the test sample contains an antibody corresponding to the target antigens, that antibody is retained on the antigen microarray by an antigen-antibody reaction. A labeled secondary antibody labeled with a fluorescent pigment is then contacted with the antigen microarray. Fluorescence of the labeled secondary antibody is then detected by a detection device such as a laser fluorescent microscope or laser scanner.

## Description

The present invention relates to a method for testing whether or not an antibody that binds with a target antigen is present in a test sample, and to an antigen microarray usable in that method. More particularly, the present invention relates to a method for determining antibody levels of human microbial pathogens.

In antibody testing methods of the prior art, after first examining a patient suffering from an infectious disease, the symptoms and infection route are investigated and the pathogen that is causing the infectious disease is predicted. Antigen corresponding to the predicted pathogen and serum collected from the patient are then allowed to react in a test tube (antigen-antibody reaction). The pathogen is then identified according to the results of the antigen-antibody reaction.

However, it was extremely difficult to narrow the candidates of the pathogen down to one to an extremely small number based on patient examination results, and the number of candidate pathogens normally ranged from several types to several tens of types. Consequently, pathogen identification was performed by testing antigen-antibody reactions for each of a comparatively large number of candidate pathogens. Since the number of tests performed increases as the number of candidate pathogens increases, considerable labor and time were required for pathogen identification. In particular, since there are cases in which all candidate pathogens considered to be the cause of patient symptoms may number up to several hundred types, it was difficult to comprehensively test these candidate pathogens without omitting any possible ones. In addition, known examples of antigen-antibody reactions include the precipitation reaction, agglutination reaction, neutralization reaction, hemolysis reaction, bacteriolysis reaction, immunoadherence reaction and immunophagocytosis. Since it is necessary to select the optimum method corresponding to the antigen from numerous antigen-antibody reactions, pathogen identification required even greater labor and time.

The objective of the present invention is to provide a method for easily and rapidly testing whether or not an antibody that binds with a target antigen is contained in a sample, and antigen microarray usable in that method. Another objective of the present invention is to provide a method for easily measuring antibody level.

To achieve the above objectives, the present invention provides a method for testing whether or not antibody that binds with a target antigen is present in a test sample. The method includes by the steps of producing an antigen microarray with spots of at least eight types of target antigens; contacting the test sample with the spots, wherein in the case where an antibody corresponding to the target antigens is contained in the test sample, the antibody is retained on the antigen microarray due to an antigen-antibody reaction; and detecting whether or not the antibody is retained on the antigen microarray.

The second aspect of the present invention provides a method for measuring blood antibody levels of microbial pathogens. The method includes the steps of producing an antigen microarray with spots of an antigen; contacting serum with the spots, wherein in the case an antibody is present in the serum, the antibody corresponding to the antigen is retained on the antigen microarray due to an antigen-antibody reaction; and detecting whether or not the antibody is retained on the antigen microarray.

Other aspects and advantages of the present invention will become apparent from the following description, taken in conjunction with the accompanying drawings, illustrating by way of example the principles of the invention.

The features of the present invention that are believed to be novel are set forth with particularity in the appended claims. The invention, together with objects and advantages thereof, may best be understood by reference to the following description of the presently preferred embodiments together with the accompanying drawings in which:
Fig. 1 is a perspective view of an antigen microarray of the present invention; and
Fig. 2 is a plan view of an antigen microarray for measurement of antibody level in the blood.

The following provides an explanation of the method for testing whether or not an antibody that binds with a target antigen is present in a test sample, and antigen microarray in accordance with a first embodiment of the present invention.

According to the method of the first embodiment, whether or not numerous types of antibodies that respectively bind with numerous types of target antigens are present in a test sample is investigated by a single testing procedure. Thus, the method of the first embodiment is preferably used for a numerous medical or molecular biological screening testing as well as for measurement of antibody level in the blood.

Examples of test samples that are used include body fluids such as serum (blood), lymph, peritoneal exudate, interstitial fluid and brain spinal fluid; culture supernatants of antibody-producing cells such as B cells and hybridomas; and antibody-containing fractions isolated and purified from body fluid or culture supernatant. A preferable test sample is serum since serum is easily collected and contains antibody at comparatively high concentrations.

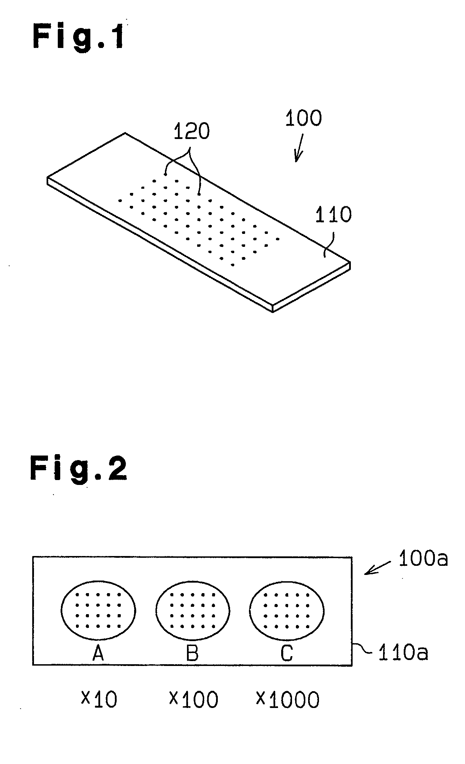

In the method of the first embodiment, whether or not a desired antibody is contained in a test sample is tested using the antigen microarray 100 shown in Fig. 1. The antigen microarray 100 has a substrate 110, such as a slide glass or a plastic plate, and spots 120 of at least eight target antigens immobilized on the upper surface of the substrate 110. The antigen microarray 100 is produced by immobilizing a plurality of target antigens 120 at different locations on the single substrate 110.

Whether or not a test sample contains an antibody that reacts with target antigens 120 is investigated by contacting the test sample with the target antigens 120 on the microarray 100.

In the method of the first embodiment, the antigen microarray 100 is used that has a plurality of types of target antigens (heteroantigens). The antigen microarray 100 is produced in the manner described below. To begin with, a plurality of types of target antigens 120 is respectively spotted at different locations on a slide glass 110. Then, the target antigens 120 are immobilized on the slide glass 110 using a known method. The antigen microarray 100 has at least eight types of target antigens 120. The number of types of target antigens 120 is preferably 10 to 1000 types, more preferably 30 to 1000 types, even more preferably 80 to 1000 types, and still more preferably 80 to 500 types. When an antigen microarray having less than 8 types of target antigens is used, the number of types of antibodies that can be detected in a single detection procedure is low, which means that a plurality of rounds of detection work is required to identify an antibody contained in a test sample. Thus, using an antigen microarray having less than 8 types of target antigens is inefficient and results in a significant increase in testing costs.

It is preferable that the antigen microarray 100 contains all known antigens. Among the known antigens, roughly 1000 types of useful antigens, and roughly 500 types of extremely useful antigens are known for use as the target antigens. Thus, at least eight types of antigens selected from useful antigens are preferably immobilized on the antigen microarray. At the current level of microarray production technology, up to 96 spots, or up to 384 spots, are typically immobilized on a single slide glass. Thus, it is economical to produce 80 to 96 spots (and more preferably 90 to 96 spots), or 300 to 384 spots (and more preferably 350 to 384 spots) on a single antigen microarray.

Preferable examples of target antigens include substances having comparatively high antigenicity such as proteins, glycoproteins and glycolipids, while more preferable examples include non-autoantigens such as endotoxin antigens and flagellar antigens of pathogens that cause infectious diseases, tumor antigens of cancer cells and allergens.

A single antigen microarray preferably has antigen spots prearranged in groups corresponding to the testing objective. For example, a single antigen microarray preferably has the spots of at least eight target antigens respectively relating to at least eight pathogens selected from a group of pathogens known to cause a single disease, a single symptom or a plurality of mutually similar symptoms.

Next, an explanation is provided of the antibody testing method. The antibody testing method includes a binding step and a detection step.

In the binding step, the test sample (single sample) is arranged on the antigen microarray. If the desired antibody is contained in the test sample, that antibody reacts with at least one of the plurality of target antigens immobilized on the antigen microarray, and is bound to the antigen microarray via that target antigen. In this case, binding between the antigen microarray and antibody is detected in the subsequent detection step. On the other hand, if the test sample does not contain an antibody having affinity for the target antigens, an antigen-antibody reaction does not occur, and the antibody does not bind to the antigen microarray. Thus, binding between the antigen microarray and antibody is not detected in the subsequent detection step.

In the detection step, whether or not an antibody is present that is bound to the antigen microarray is detected. This detection uses known detection means that can be applied to a microarray method. For example, a labeled secondary antibody can be used that has been labeled with a fluorescent pigment such as Cy3 or Cy5 having high detection sensitivity and which can be handled easily. Labeled secondary antibody specifically binds with the constant region (such as an Fc fragment) of the immunoglobulin (Ig) of animal species from which the sample was collected. The labeled secondary antibody is preferably an anti-IgG antibody or anti-IgM antibody.

The antibody detection method of the present invention can be applied to the measurement of blood antibody level. In the case of measuring blood antibody level, serum is diluted to different dilution factors (for example, serially in the manner of 10 times, 100 times and 1000 times) to prepare a plurality of test sample liquids (serum dilution series). Each of the plurality of test sample liquids are arranged on the antigen microarray and whether or not they react with the target antibodies immobilized on the antigen microarray is investigated using the procedure described above. The resistance or sensitivity of a patient to a disease such as an infectious disease, cancer or allergy is then predicted from the results of measuring blood antibody level. Since the antigen microarray has at least eight target antigens, antibody level can be measured simultaneously for 8 or more types of antibodies.

In measuring blood antibody level, an antibody microarray (single array) is used on which a plurality of target antigens is respectively spotted at a plurality of locations. More specifically, a single target antigen is spotted at a plurality of locations on a single antigen microarray at the same concentration and in the same amount. It is economically preferable to use a single antigen microarray for testing a single serum dilution series. In this case, testing is conducted in order starting with the test sample liquid having the highest dilution factor. In addition, a single antigen microarray may be used for testing a single test sample liquid. In this case, since a plurality of test sample liquids having mutually different concentrations can be tested simultaneously (in parallel), the amount of time required for measuring blood antibody level is shortened.

The antibody testing method is used to identify pathogens, tumor antigens, allergens and so forth that are the cause of diseases including infectious diseases, cancer, allergies and the like (diseases associated with activation of the immune system). In addition, the antibody testing method is used to predict the sensitivity or resistance of a patient (human or mammal) to these diseases.

In the case of identifying the cause of an infectious disease, cancer or allergy, a physician or other health professional predicts the pathogen, tumor antigen or allergen based on symptoms, and then selects an antigen microarray having an antigen corresponding to the predicted pathogen, tumor antigen or allergen, namely the target antigen. Preferably, an antigen microarray is selected that has spots of target antigens derived from all of the predicted pathogens, cancer cells or allergens. It should be noted that if there are frequently diagnosed diseases, a plurality of antigen microarrays may be prepared in advance in accordance with the testing pattern for diagnosing those diseases.

Next, serum or other test sample is collected from the patient. The test sample is contacted with the selected antigen microarray, and whether or not an antibody that binds with the target antigens is present in the test sample is investigated. More specifically, the test sample is first placed on the antigen microarray. The antigen microarray is arranged under conditions so as to inhibit non-specific reactions and induce specific antigen-antibody reactions. In the case there is an antibody corresponding to a target antigen on the antigen microarray, that antibody binds to the target antigen and is retained on the antigen microarray.

Test sample that has been retained on the antigen microarray is removed and the antigen microarray is washed gently. A labeled secondary antibody is then placed on the antigen microarray. In the case the antibody is retained on the antigen microarray, the labeled secondary antibody binds to the antibody and as a result, is retained on the antigen microarray.

The labeled secondary antibody that was not retained on the antigen microarray is removed, and the antigen microarray is washed gently. The labeled secondary antibody retained on the antigen microarray is then detected using a detection device such as a laser fluorescent microscope, and laser scanner. The detection device is preferably able to indicate the presence of labeled secondary antibody visually.

If a predicted antibody, namely an antibody corresponding to the target antigens of the antigen microarray, is not present in the test sample, the antigen microarray is not visualized by the detection device. On the other hand, if a predicted antibody is present in the test sample, since specific binding sites of that antibody are specifically bound with target antigens on the antigen microarray, and labeled secondary antibody is bound to the constant region of that antibody, labeled secondary antibody bound to that antibody is visualized by the detection device. Target antibodies are then identified and the presence of corresponding antibody is then confirmed based on the locations of the visualized spots of the antigen microarray. For example, in the case the label is a fluorescent substance, the detection device detects the fluorescent substance and target antigens are identified from those locations. In this manner, a pathogen, tumor antigen or allergen present in the body of a patient is identified by testing for the presence of antibody in a test sample that specifically binds with a target antigen.

The blood antibody levels of microbial pathogens are determined by modifying the antibody testing method. The following provides an explanation of a method for measuring antibody level. To begin with, serum collected from a patient is serially diluted to prepare a plurality of sample liquids having mutually different concentrations (serum dilution series). Each sample liquid is placed on a single antigen microarray. The binding and detection steps are carried out in the same manner as described above. By carrying out the binding and detection steps for a plurality of concentrations of sample liquids, the dilution factor of the sample liquid having the highest dilution factor that reacts with an antigen of the antigen microarray is obtained. The blood antibody level is then calculated from the inverse of the resulting dilution factor.

The following advantages are obtained according to the first embodiment.

In the antibody testing method of the first embodiment, an antigen microarray is used having spots for at least eight types of target antigens. The test sample is simultaneously contacted with at least eight types of target antigens. Thus, since an antigen microarray is used that has extremely high detection sensitivity and allows large-volume screening, the presence of antibody to numerous types of target antigens can be tested both easily and rapidly. Since whether or not an antibody is bound to the target antigens can be detected in an extremely short time, pathogens and tumor antigens can be identified in a short period of time. In particular, an antigen microarray having spots of all known antigens as the target antigen 120 is preferable for detecting or identifying pathogens and tumor antigens in short time. Thus, the present invention is extremely useful in medical fields such as the diagnosis of diseases and treatment of patients.

Since the presence of numerous types of antibodies can be tested with a single detection routine, screening testing can be conducted efficiently. The predicted cause of an infectious disease, cancer or allergy can be determined in nearly a single detection routine. Although infectious diseases have become increasingly diversified in recent years, making their diagnosis more difficult, according to this antibody testing method, identification of the cause of infectious diseases that are difficult to predict is extremely easy.

Since there was an extremely large number of means for visualizing and detecting the results of. the antigen-antibody reaction, the selection of detection means and detection work conventionally required considerable labor. In contrast, in the first embodiment, the results of antigen-antibody reactions are detected simply by visualizing a labeled secondary antibody on the antigen microarray. Thus, detection is extremely easy and requires little labor.

The presence of primary antibody is detected by amplifying by using a labeled secondary antibody. Thus, detection sensitivity is comparably high.

In the antibody testing method of the first embodiment, processing work of test samples is nearly constant regardless of the type of test sample or type of antigen microarray. Thus, workers are able to easily become familiar with testing, thereby decreasing the likelihood of errors and confusion. Since the tabulation of test results, which is particularly susceptible to the occurrence of human error, is not required, the antibody testing method of the first embodiment is useful in medical fields having a comparatively high level of reliability. In addition, since processing involves routine work, the efficiency of antibody detection can be improved easily. For example, antibody detection can be carried out by an antibody detection device programmed with the antibody testing method of the first embodiment.

Since the test sample is in the form of a series of serum dilutions, blood antibody level can be measured easily and rapidly. By combining antibody detection and measurement of blood antibody level in particular, the presence of numerous types of antibodies can be detected while simultaneously measuring blood antibody level with respect to the detected antibodies in a single procedure, thereby making this extremely useful.

Since the antigen microarray is spotted with endotoxin antigens, flagellar antigens, tumor antigens or allergens, the causes of infectious diseases, cancer or allergies are identified rapidly and easily. In the case of using an antigen microarray having endotoxin antigens or flagellar antigens in particular, such an antigen microarray is useful for patient treatment since the resistance and sensitivity of the patient to infectious diseases is tested easily and rapidly. In the case of using an antigen microarray having various tumor antigens, cancers can be detected in early stages. In the case of using an antigen microarray having various allergens, the sensitivity of a patient or allergic person to the allergens is tested easily.

Since a plurality of antigens derived from a plurality of pathogens that have been correlated with the objective of testing (for example, a disease or symptoms) are immobilized on the antigen microarray, an antigen that is the cause of a disease is identified comprehensively and easily without omitting any possible causative antigens. If various types of antigen microarrays are prepared in advance, in the case, for example, an emergency situation has occurred, an unknown antigen can be identified rapidly, which is useful in rapidly handling the emergency situation.

If a single antigen microarray is prepared corresponding to a single symptom, a physician can easily identify an antigen by selecting an antigen microarray corresponding to the symptom, contacting a test sample with the selected antigen microarray, and detecting the antigen for which an antigen-antibody reaction has occurred.

The following provides an explanation of an antibody testing method and antigen microarray according to a second embodiment of the present invention while focusing on the differences with the first embodiment. In the second embodiment, each of a plurality of types of antigens are respectively immobilized at a plurality of locations and at a plurality of concentrations. In other words, a single target antigen is spotted at a plurality of locations on the antigen microarray, and the concentrations of the target antigen at the plurality of spots are mutually different. The antibody testing method of this second embodiment is suited for measurement of antibody level in the blood. Since the antigen microarray of the second embodiment is spotted with at least eight types of target antigens, the presence of antibodies to numerous types of target antigens is detected with a single procedure, and the blood antibody levels of the detected antibodies are also measured. Furthermore, less than 8 types of target antigens may be spotted for a single target antigen.

When measuring blood antibody level, serum is first collected from the patient suffering from a disease such as an infectious disease, cancer or allergy. The serum is placed on the antigen microarray, and binding and detection steps are carried out in the same manner as the first embodiment. At this time, a sample liquid prepared by diluting the serum may also be used as necessary.

In the case the serum (or its diluted sample liquid) is retained on the antigen microarray, a spot of the target antigen is visualized in the detection step. In this case, spots of target antigens having a low concentration may not be visualized. Thus, the detection limit concentration is determined from the location of the target antigen having the lowest concentration among those spots of the target antigens that are visualized. The blood antibody level is then calculated from that detection limit concentration. Alternatively, the concentration of a labeled secondary antibody retained on the antigen microarray may be measured using a measurement detection device such as a laser scanner. In this case, a graph is produced that indicates the fluorescent intensity of each spot and the concentrations of target antigens, after which blood antibody level is calculated from the graph. Thus, blood antibody level is measured with greater precision.

The following advantages are obtained according to the second embodiment.

Since an antigen microarray is used having a plurality of spots of the same type of antigen at serially different concentrations, the labor of preparing a serum dilution series can be eliminated. In addition, the reaction between serum and the antigen microarray is carried out once, thereby eliminating the need to repeat the procedure for a plurality of diluted sample liquids. Thus, measurement of blood antibody level is carried out efficiently in a short time.

Since the antigen microarray has spots of a plurality of types of target antigens, whether or not a plurality of types of antibodies is present in serum can be confirmed with a single procedure.

In the case of conventional measurement of antibody level in antiserum, a fixed amount of antigen was added in a test tube to respective sample liquids of a series of antiserum dilutions. The dilution factor of the antiserum was determined at the endpoint of a reaction such as neutralization, agglutination or precipitation, and antibody level was calculated from the inverse of the dilution factor. However, there are various forms of antigen-antibody reactions, including a precipitation reaction, agglutination reaction, neutralization reaction, hemolysis reaction, bacteriolysis reaction, immunoadherence reaction and immunophagocytic action. Consequently, a method and means for visualizing whether or not an antigen-antibody reaction has occurred has not been established in the prior art, making this bothersome and inefficient. In addition, depending on the particular visualization method or means, the detection sensitivity was excessively low, making these unsuitable for measurement of antibody level. In contrast, in the second embodiment, blood antibody level is measured using single detection means of detecting labeled secondary antibody on an antigen microarray. Thus, the level of the target antibody contained in serum is measured extremely easily and rapidly.

The following provides an explanation of embodiments of the present invention.

### [Investigation of Antibody Patterns of Normal Adults]

The corresponding gene products (endotoxin antigens, flagellar antigens and adventitial tumor protein antigens) were respectively purified from the pathogens listed in Table 1. The purified gene products were spotted and immobilized on a single slide glass to prepare an antigen microarray.

**Table 1**

| List of Depicted Target Antigens Immobilized on Antigen Microarray | |
|---|---|
| Pathogens | Antigens |
| Aeromonas hydrophila | Enterotoxin gene product, OMP |
| Bacillusn anthracis | Pag, PA, EF |
| Bacillus cereus | Haemolysin |
| Bacteroides fragilis | Enterotoxin, LPS, OMP, Flagellin |
| Bordetella pertussis | PTX, Fha , Fim |
| Borrelia burgdorferi | OspABC, Flagellin |
| Burkholderia cepacia | LPS, OMP, Flagellin, Alpase |
| Burkholderia pseudomallei | LPS, OMP, Flagellin, Alpase |
| Chlamydia trachomatis | MOPS, DnaJK, GroEL, OMP |
| Chlamydia psittasi | OMP, MOPS, DnaJK, GroEL |
| Chlamydia pneumoniae | OMP, MOPS, DnaJK, GroEL |
| Clostridium difficile | Toxin A, Toxin B |
| Clostridium perfringens | Alfa toxin, Beta toxin, theta-toxin |
| Clostridium tetani | Tetanospasmin |
| Clostridium septicum | alfa toxin, delta toxin |
| Corynebacerium diptheriae | diphtheria toxin |
| Coxiella burnetii | immunoreactive antigen,DnaJK, GroEL, OMP |
| Ehrlichia phagocytophila | DnaJK, GroEL, OMP |
| Escherichia coli | InvE, Intimin, Shiga toxin, ST, LT, LPS, Flagellin |
| Enterococcus spp. | Cytolysin |
| Francisella tularensis | GroEL, DnaJK, AcpA, TUL, Fop, LPS |
| Haemophilus influenzae | LPS, OMP |
| Helicobacter pylori | Cag, LPS |
| Legionella pneumophila | LPS, Omp, Mip, DotB, Metaloprotease |
| Listeria monocytogenes | Lysteriosin, Hemolysin, Flagellin |
| Leptospira interrogans | Flagellin, OMP |
| Pseudomonas aeruginosa | LPS, Omp, Flagellin, ExoA, PilA |
| Orientia tsutsugamushi | GroEL, DnaJK, Omp |
| Mycobacterium tuberculosis | GroEL, DnaJK |
| Salmonella spp. | LPS, Omp, Enterotoxin, FliC, InvAB, SipBC |
| Serratia marcescens | LPS, Omp |
| Salmonella typhi | Vi, FliC, Enterotoxin, Omp, InvAB, SipBC |
| Staphylococcus aureus | ETA, ETB, SEA,SEB, SEC, SED,SEE, TSST |
| Streptococcus pyogenes | SLO, SLS, M antigen ,T antigen, SpeABC, SagAC, SSA |
| Streptococcus pneumoniae | Ply, LytA |
| Treponema pallidom | 47kD antigen, OMP |
| Vibrio parahaemolyticum | LPS, Omp TdH |
| Vibrio cholerae | Omp, LPS, Flagella, CT, Zot, Pili |
| Yersinia pestis | ST, Pst, YopE, YadA, Invasin, Omp |
| Influenza virus | HA, Neuramidase |
| Adenovirus | Major capsid proteins |
| CM virus | Major envelope proteins |
| Japanese encephalitis virus | Major envelope proteins |

Next, serum was collected from normal adults (Japanese). The serum was serially diluted by a factor of 10 to prepare a plurality of serum sample liquids having different dilution factors. Testing was carried out in the manner described below in order starting with the serum liquid sample that was diluted the greatest, namely the serum liquid sample having the highest dilution factor.

To begin with, the serum sample liquid having the highest dilution factor was placed on the antigen microarray. Free serum components that were not retained on the antigen microarray were removed from the antigen microarray, and the antigen microarray was washed gently. Labeled secondary antibody (Cy5-labeled anti-human IgG and IgM antibodies) were contacted with the antigen microarray. Whether or not the labeled secondary antibody, and more specifically, the Cy5-labeled antibody, is retained on the antigen microarray was investigated using a laser scanner for microarrays. The locations of the fluorescent spots generated by the Cy5 label were stored in the memory of a computer.

The antigen microarray was washed well to remove serum components and secondary antibody from the antigen microarray. Similar testing was carried out for the test sample liquid having the second highest dilution factor (namely the second most diluted sample liquid). The above procedure was then carried out in order starting from the test sample liquid having the highest dilution factor. The obtained results were tabulated with a computer, and in addition to identifying the types of antibodies contained in the serum, the blood levels of those antibodies were determined.

Similar tests were carried out on a plurality of normal adults. In this manner, the average antibody pattern of normal adults along with a database of blood antibody levels were produced and stored in a computer.

The first and second embodiments may be changed in the manner described below.

An antigen microarray may also be produced by spotting antigens related to various groups of diseases (endotoxin antigens of various pathogens, flagellar antigens, tumor antigens and allergens) on a single slide glass. In this case, a wide range of diseases are tested in a single round of testing.

Serum of a large number of normal adults may be tested using an antigen microarray spotted with numerous types (about 500 types or about 1000 types) of target antigens, and the average antibody pattern may be recorded and used for diagnosis. In this case, by testing patient serum with the same type of antigen microarray and comparing those results with a normal adult antibody pattern, characteristic antibodies can be easily detected in the patient serum, thereby making this useful for treatment.

Examples of labeled secondary antibodies that can be used include secondary antibodies bound with an enzyme such as alkaline phosphatase or peroxidase.

The antibody testing method of the present invention can also be used for screening during production of polyclonal antibodies or monoclonal antibodies.

In the second embodiment, instead of producing an antigen microarray having a plurality of spots of the same type of antigen at serially different concentrations, an antigen microarray may be produced having long, narrow, linear spots of the same type of antigen for which concentration is changed consecutively. In this case, blood antibody level is measured more precisely.

In the second embodiment, an antigen microarray 100a shown in Fig. 2 can be used. The antigen microarray 100a is preferable for measurement of antibody level in the blood. In detail, the antigen microarray 100a includes a plurality of regions A, B and C defined on a substrate 110a. Spots of a plurality of types of target antigens are immobilized on each of the regions A, B and C. In the case of measuring blood antibody level, serum is diluted to different dilution factors (for example, serially in the manner of 10 times, 100 times and 1000 times) to prepare a plurality of test sample liquids (serum dilution series). For example, 10-times diluted test sample liquid is arranged on the region A, 100-times diluted test sample liquid is arranged on the region B, and 1000-times diluted test sample liquid is arranged on the region C. Since the test samples are reacted with the target antibodies spotted in each of the regions A, B and C, antibody levels can be measured simultaneously for a plurality types of antibodies.

It should be apparent to those skilled in the art that the present invention may be embodied in many other specific forms without departing from the spirit or scope of the invention. Therefore, the present examples and embodiments are to be considered as illustrative and not restrictive and the invention is not to be limited to the details given herein, but may be modified within the scope and equivalence of the appended claims.

## Claims

1. A method for testing whether or not antibody that binds with a target antigen is present in a test sample, the method **characterized by** the steps of:
producing an antigen microarray with spots of at least eight types of target antigens;
contacting the test sample with the spots, wherein in the case where an antibody corresponding to the target antigens is contained in the test sample, the antibody is retained on the antigen microarray due to an antigen-antibody reaction; and
detecting whether or not the antibody is retained on the antigen microarray.

2. The testing method according to claim 1, wherein the step of producing the antigen microarray includes immobilizing the spots of 10 to 1000 types of target antigens on the antigen microarray.

3. The testing method according to claim 1, wherein the test sample is a plurality of serum diluted liquids that have been diluted to different dilution factors, and the testing method further includes calculating the blood antibody level of the antibody using the results of the detection step.

4. The testing method according to claim 1, wherein the step of producing the antigen microarray includes immobilizing a plurality of spots having serially different concentrations on the antigen microarray for each of the target antigens, and the testing method further includes calculating the blood antibody level of the antibody using the results of the detection step.

5. The testing method according to claim 1, wherein each of the target antigens is an endotoxin antigen, flagellar antigen, tumor antigen or allergen.

6. The testing method according to claim 1, further includes selecting the at least eight types of target antigens from a prescribed antigen group that causes the disease being tested.

7. The testing method according to claim 1, wherein the detection step includes contacting labeled secondary antibody with the antigen microarray, and detecting whether the labeled secondary antibody is retained on the antigen microarray.

8. The testing method according to claim 8, wherein the step of detecting the labeled secondary antibody includes visualizing the labeled secondary antibody retained on the antigen microarray.

9. A method for measuring blood antibody level **characterized by** the steps of:
producing an antigen microarray with spots of an antigen;
contacting serum with the spots, wherein in the case an antibody is present in the serum, the antibody corresponding to the antigen is retained on the antigen microarray due to an antigen-antibody reaction; and
detecting whether or not the antibody is retained on the antigen microarray.

10. The measurement method according to claim 9, wherein the step of producing the antigen microarray includes forming spots of the antigen having serially different concentrations on the antigen microarray.

11. The measurement method according to claim 9, wherein the step of producing the antigen microarray includes spotting a spot of the antigen having consecutively grading concentrations on the antigen microarray.

12. The measurement method according to claim 9, wherein the antigen is one of eight types of antigens.

13. The measurement method according to claim 9, further comprising calculating blood antibody level of the antibody using the results of the detection step.

14. The antigen microarray (100; 100a) usable in the method according to any one of claims 1 to 13, **characterized by**:
a substrate (110; 110a), and
spots (120) of at least eight types of target antigens immobilized on the substrate.
